(19) Europäisches Patentamt
European Patent Office
Office européen des brevets

(11) Numéro de publication: **0 007 821**
**B1**

(12) # FASCICULE DE BREVET EUROPEEN

(45) Date de publication du fascicule de brevet: **18.03.81**

(51) Int. Cl.³: **A 61 K 31/485** // (A61K31/485, 31/445)

(21) Numéro de dépôt: **79400422.6**

(22) Date de dépôt: **26.06.79**

(54) **Nouvelle composition analgésique.**

(30) Priorité: **13.07.78 FR 7820969**

(43) Date de publication de la demande: **06.02.80 Bulletin 80/3**

(45) Mention de la délivrance du brevet: **18.03.81 Bulletin 81/11**

(84) Etats contractants désignés: **BE CH DE FR GB IT LU NL SE**

(56) Documents cités: **FR-A-2 334 358**

(73) Titulaire: **PHARMINDUSTRIE, 35 Quai du Moulin de Cage, F-92231 Gennevilliers (FR)**

(72) Inventeur: **Uzan, André Victor, 35, Avenue Victor-Hugo, F-75016 Paris (FR)**

(74) Mandataire: **Houssin, Jean Produits Chimiques Ugine Kuhlmann et al, Service Propriété Industrielle Tour Manhattan Cedex 21, F-92087 Paris la Defense (FR)**

**EP 0 007 821 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouvelle composition analgésique

La présente invention a pour objet une composition analgésique utilisable lors du traitement des états hyperalgiques.

La thérapeutique actuelle des états hyperalgiques repose sur deux types de produits:

- les uns, comprenant les anti-inflammatoires non stéroidiens, doivent leur activité à des effets essentiellement périphériques.
- les autres, comprenant le groupe des opiacés et notamment la morphine, doivent leur activité à un effet purement central.

Les récents progrès réalisés dans la connaissance du mécanisme d'action des analgésiques morphiniques ont mis en évidence l'intervention des neuromédiateurs de structure peptidique et de leurs récepteurs neuronaux centraux contrôlant la douleur.

Ces données récentes n'excluent cependant pas le rôle, par ailleurs bien établi, des régulations tryptaminergiques centrales sérotonino-dépendantes, dans la modularion des états d'hyperalgie ou d'analgésie.

Ces deux mécanismes en cause ne s'opposent pas, mais semblent, bien au contraire, complémentaires.

Le rôle de la sérotonine (5-HT) dans la sensibilité à des stimuli douloureux est bien admis. Ainsi par exemple, la lésion cérébrale des neurones tryptaminergiques ou la diminution en 5-HT entraînent une augmentation de la réponse douloureuse à un stimulus électrique (Harvey, J.A. & coll., Sciences, 148, 250 [1965]).

Il apparaît donc, à la lumière de ces données bibliographiques, que l'administration d'une substance prosérotoninergique, lors d'une thérapeutique analgésique à l'aide d'un morphinique par exemple, est susceptible d'en améliorer les effets.

Une telle synergie paraît particulièrement souhaitable car elle pourrait prendre le relais des traitements morphiniques ou réduire en posologie et en durée ces traitements par un effet de potentialisation.

Connaissant les effets indésirables que provoquent les opiacés et les risques d'accoutumance auxquels ils exposent, l'intérêt d'une alternance ou d'une synergie est bien évident. Cependant, peu de substances sont réellement cepables d'intensifier les fonctions neuronales de la 5-HT cérébrale.

Les précurseurs de 5-HT tels que le 5-HTP ou tryptophane doivent être administrés à doses très élevées et malgré cela leur efficacité est discutable et leurs effets secondaires non négligeables.

Les inhibiteurs de recapture de 5-HT sont donc à priori susceptibles de manifester des propriétés prosérotoninergiques appréciables. Cependant, jusqu'à présent, leur activité et leur spécificité sont restées insuffisantes, ce qui expliquerait l'absence d'activité analgésique et l'absence de synergie avec la morphine, de la chlorimipramine. Dans le cas de cette substance du groupe des tricycliques, pourtant la plus spécifique de ce groupe des tricycloques, pourtant la plus spécifique de ce groupe vis-à-vis de la 5-HT, l'inefficacité peut être expliquée par sa transformation dans l'organisme en chlordésipramine dont l'activité porte principalement sur l'inhibition de la Recapture de noradrénaline.

Or il a été trouvé, conformément à la la présente invention, qu'une composition comprenant la morphine ou un dérivé morphinique tel que la péthidine et un inhibiteur très spécifique et très actif de recapture neuronal et plaquettaire de la sérotonine de formule (I) présente de très fortes propriétés analgésiques.

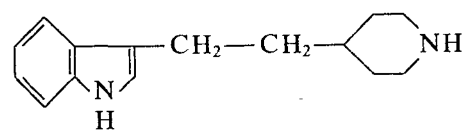

(I)

Selon la nature et le type des dérivés morphiniques utilisés, la proportion en poids des dérivés morphiniques dans la composition varie de 0,1 à 10 fois le poids du composé de formule (I). Ainsi, dans le cas de la morphine cette proportion en poids varie de 0,25 à 0,5 fois le poids du composé de formule (I) et dans le cas de la péthidine, elle varie de 1 à 2 fois le poids du composé de formule (I).

L'activité analgésique de la morphine est potentialisée par le composé de formule (I) alors que la chlorimipramine, autre inhibiteur de recapture de la 5-HT, ne la fait pas.

Il est donc permis de penser que le composé de formule (I) bénéficie de ces propriétés sans doute en tant qu'inhibiteur puissant et spécifique de la recapture de la 5-HT (Le Fur et al. Biochem. Pharmacol. 26, 497, 1977) mais peut être aussi, grâce à un effet cérébral régional propre, au niveau de certains récepteurs.

Le composé de formule (I) est connu et a été décrit notamment dans la demande de brevet FR-A-2 334 358 déposée le 12 Décembre 1975.

## Propriétés pharmacologiques

La potentialisation de l'activité analgésique de la morphine par le composé de formule (I) est mise en évidence expérimentalement chez le rat et la souris par la mesure du réflexe antinociceptif provoqué chez l'animal lorsqu'il est placé sur une plaque chauffante.

### 1) Etude chez le rat

La méthode utilisée est celle de Sugrue & coll. [J. Pharm. Pharmacol., 28, 447 (1976)].

#### Protocole expérimental

Des rats mâles (lots de 8 animaux) en provenance de l'élevage Charles River, pesant de 70 à 90 g, reçoivent une injection intrapéritonéale, soit du composé de formule (I) (20 mg/kg), soit de 5-HTP (150 mg/kg), soit de chlorimipramine (20 mg/kg), soit de sérum physiologique pour les témoins. Trente minutes apès, ils reçoivent une seconde injection, soit de morphine (voie sous-cutanée, 4 mg/kg), soit de sérum physiologique. Au bout de trente minutes, chaque rat est placé individuellement sur une plaque chauffée à 55°C. On note alors le temps d'apparition du réflexe antinociceptif. Le temps maximum d'exposition est de 60 secondes.

#### Résultats

Les résultats obtenus sont rassemblés dans le tableau I.

Ils montrent que dans les conditions expérimentales décrites le composé de formule (I), le 5-HTP et la chlorimipramine, ne présentent pas chez le rat d'effet analgésique propre.

Cependant, le composé de formule (I) potentialise de façon significative l'activité analgésique de la morphine en doublant pratiquement le temps d'apparition du réflexe antinociceptif par rapport aux animaux recevant la morphine seule.

Le 5-HTP et la chlorimipramine conduisent à des augmentations non significativement différentes de celles données par la morphine seule.

### 2) Etude chez la souris

La méthode utilisée est celle de Jacob & coll. [Arch. Int. Pharmacodyn., 133, 296 (1961)].

#### Protocole expérimental

Des souris mâles (élevage Charles River) pesant 24 à 28 g (lots de 8 animaux) reçoivent une injection intrapéritonéale, soit du composé de formule (I) (20 mg/kg), soit de produits de comparaison: 5-HTP (150 à 200 mg/kg), chlorimipramine, désipramine (20 mg/kg), soit de sérum physiologique pour les témoins. Trente minutes après, les animaux reçoivent une injection de morphine (voie sous-cutanée, 8 mg/kg), de nalorphine (voie intrapéritonéale, 50 mg/kg) ou de sérum physiologique. Trente minutes après, chaque souris est placée sur une plaque chauffée à 65°C. On note le temps d'apparition du réflexe antinociceptif. On procède à trois expositions successives sur la plaque chauffante, le temps maximum de chaque exposition étant de 60 secondes.

#### Résultats

Les résultats sont rassemblés dans le tableau II.

On constante alors que dans cette épreuve la morphine allonge dans une proportion importante le temps d'apparition du réflexe, cet effet étant maximum lors de la 3ème exposition sur la plaque chauffante.

La nalorphine est sans efft, comme on pouvait s'y attendre.

Le composé de formule (I) et le 5-HTP exercent chez la souris une légère action analgésique propre à la dose de 20 mg/kg (voie intrapéritonéale). La chlorimipramine et la désipramine sont sans effet.

L'action propre du composé de formule (I) et du 5-HTP est antagonisée par la nalorphine puisque lorsqu'ils sont associés à ce produit on ne retrouve plus leur léger effet propre.

En revanche, le composé de formule (I) et le 5-HTP potentialisent très nettement l'action analgésique de la morphine aussi bien lors de la 1ère que de la 2ème et surtout de la 3ème exposition.

La chlorimipramine ne modifie pas l'activité de la morphine dans les mêmes conditions.

La potentialisation de la morphine par le composé de formule (I) est importante puisque, à la 3ème exposition, le temps d'apparition du réflexe antinociceptif de la souris, qui était déjà quadruplé par la morphine seule, est encore plus prolongé par association avec le composé de formule (I), les temps devenant dans ce cas 5 à 10 fois supérieurs à ceux des témoins.

TABLEAU I

ACTIVITE ANALGESIQUE
Chez le rat

| PRODUITS | DOSE mg/kg | VOIE | Temps d'apparition en secondes par rapport aux témoins du seuil douloureux | |
|---|---|---|---|---|
| Témoins | — | | 8,2 | |
| MORPHINE | 4 | SC | 37,5 | ++ |
| 5-HTP | 150 | IP | 9,9 | NS |
| Composé de formule (I) | 20 | IP | 9,5 | NS |
| CHLORIMIPRAMINE | 20 | IP | 13,5 | NS |
| MORPHINE+5-HTP | 4<br>150 | SC<br>IP | 41,2 | NS |
| MORPHINE+<br>Composé de formule (I) | 4<br>20 | SC<br>IP | 58,9 | ++ |
| MORPHINE+<br>CHLORIMIPRAMINE | 4<br>20 | SC<br>IP | 51,2 | NS |

++ $0,001 < p < 0,01$
NS non significatif

TABLEAU II

ACTIVITE ANALGESIQUE
Chez la souris

| PRODUIT | VOIE | DOSE mg/kg | TEMPS D'APPARITION DU REFLEXE ANTINOCICEPTIF EN SECONDES 1re exposition | 2è exposition | 3è exposition |
|---|---|---|---|---|---|
| Témoins | — | — | 4,7—6,5 | 6,6—7,6 | 3,5—5 |
| MORPHINE | SC | 8 | 15,7 | 16,5 | 20 |
| NALORPHINE | IP | 50 | 7,9 | 6,6 | 3,9 |
| 5-HTP | IP | 150<br>200 | 11,2<br>11,5 | 19,5<br>10,9 | 12,4<br>5,8 |
| CHLORIMIPRAMINE | IP | 20 | 7,5 | 6,5 | 3,1 |
| DESIPRAMINE | IP | 20 | 7 | 8,7 | 7 |
| COMPOSE DE FORMULE (I) | IP | 20 | 8,7—10,4 | 10—13,2 | 5,7—5,9 |
| MORPHINE+<br>CHLORIMIPRAMINE | SC<br>IP | 8<br>20 | 11,2 | 11,9 | 12,6 |
| MORPHINE+<br>5-HTP | SC<br>IP | 8<br>200 | 42,5 | 51 | 54,4 |
| MORPHINE+<br>DESIPRAMINE | SC<br>IP | 8<br>20 | 9 | 13,3 | 12,9 |
| MORPHINE+<br>COMPOSE DE FORMULE (I) | SC<br>IP | 8<br>20 | 31,6 | 40 | 48,7 |
| NALORPHINE+<br>COMPOSE DE FORMULE (I) | IP<br>IP | 50<br>20 | 9,2 | 6,9 | 6,1 |
| NALORPHINE+<br>5-HTP | IP<br>IP | 50<br>150 | 5,4 | 3,6 | 4,1 |

L'effet analgésique de la composition selon l'invention est mis en évidence expérimentalement chez la souris.

Protocole expérimental

Des souris CD1, pesant de 25 à 29 g reçoivent la composition selon l'invention soit par voie sous-cutanée soit par voie intrapéritonéale suivant le dérivé morphinique utilisé. Trente minutes après, elles subissent l'épreuve de la plaque chauffante portée à 65°C, selon Barthélémy & coll. [J. Pharmacol. (Paris), 2, 35 (1971)] (troi expositions successives à 5 minutes d'intervalle, temps maximum d'exposition 60 secondes). On note le temps d'apparition du réflexe antinociceptif (léchage simultané des pattes antérieures, saut ou bond ajusté).

Résultats

Le tableau III montre que le composé de formule (I), administré en même temps que la morphine ou la péthidine, potentialise l'effet analgésique de ces deux produits.

Aux doses utilisées, 30 mg/kg sous-cutanée et 20 mg/kg intrapéritonéale, le composé de formule (I) provoque un effet analgésique très faible et non significatif.

La morphine, à 8 mg/kg sous-cutanée, provoque un effet significativement différent des témoins, aux trois expositions. L'analgésie est importante à la 3ème exposition, ce qui correspond à un effet spécifique des morphiniques selon Barthélémy & coll. L'effet de la morphine est potentialisé dans la composition et l'activité est particulièrement importante et significative à la 3ème exposition.

TABLEAU III

ACTIVITE ANALGESIQUE DE LA COMPOSITION

Chez la souris

| PRODUITS | DOSES | TEMPS D'APPARITION DU REFLEXE ANTINOCICEPTIF EN SECONDES 1ère EXPOSITION | | | | |
|---|---|---|---|---|---|---|
| | mg/kg | Moyenne S±SE | Différence par rapport aux témoins | | Différence par rapport à l'analgésique | |
| | VOIES | | P | Effet % | P | Effet % |
| TEMOINS | Sol. 9º/oo NaCl I.P. | 5,7±1,2 | — | — | — | — |
| MORPHINE | 8 S.C. | 13,2±2,8 | 0,05 | 234 | — | — |
| COMPOSE DE FORMULE (I) | 30 S.C. | 8,9±2,3 | NS | 154 | — | — |
| MORPHINE+ COMPOSE DE FORMULE (I) | 8 S.C. 30 S.C. | 24,1±7,1 | 0,05 | 419 | 0,20 | 182 |
| PETHIDINE | 40 I.P. | 9,5±1,1 | 0,10 | 160 | — | — |
| COMPOSE DE FORMULE (I) | 20 I.P. | 7,6±0,9 | NS | 132 | — | — |
| PETHIDINE+ COMPOSE DE FORMULE (I) | 40 I.P. 20 I.P. | 30,8±8,6 | 0,02 | 535 | 0,05 | 332 |

TABLEAU III (SUITE)

ACTIVITE ANALGESIQUE DE LA COMPOSITION

Chez la souris

| PRODUITS | DOSES | TEMPS D'APPARITION DU REFLEXE ANTINOCICEPTIF EN SECONDES 2ème EXPOSITION | | | | |
|---|---|---|---|---|---|---|
| | mg/kg | Moyenne S±SE | Différence par rapport aux témoins | | Différence par rapport à l'analgésique | |
| | VOIES | | P | Effet % | P | Effet % |
| TEMOINS | Sol. 9º/oo NaCl I.P. | 6,8±1,1 | — | — | — | — |
| MORPHINE | 8 S.C. | 11,2±1,2 | 0,02 | 164 | — | — |
| COMPOSE DE FORMULE (I) | 30 S.C. | 8,7±1,6 | NS | 127 | — | — |
| MORPHINE+ COMPOSE DE FORMULE (I) | 8 S.C. 30 S.C. | 34,7±9,2 | 0,01 | 510 | 0,05 | 308 |
| PETHIDINE | 40 I.P. | 12,7±3,9 | 0,20 | 186 | — | — |
| COMPOSE DE FORMULE (I) | 20 I.P. | 8,5±2,2 | NS | 125 | — | — |
| PETHIDINE+ COMPOSE DE FORMULE (I) | 40 I.P. 20 I.P. | 23,6±8,1 | 0,10 | 347 | NS | 185 |

TABLEAU III (SUITE ET FIN)

ACTIVITE ANALGESIQUE DE LA COMPOSITION
Chez la souris

| PRODUITS | DOSES mg/kg VOIES | TEMPS D'APPARITION DU REFLEXE ANTINOCICEPTIF EN SECONDES 3ème EXPOSITION Moyenne S±SE | Différence par rapport aus témoins P | Effet % | Différence par rapport à l'analgésique P | Effet % |
|---|---|---|---|---|---|---|
| TEMOINS | Sol. 9⁰/oo NaCl I.P. | 4,1±0,8 | — | — | — | — |
| MORPHINE | 8 S.C. | 11±1,8 | 0,01 | 268 | — | — |
| COMPOSE DE FORMULE (I) | 30 S.C. | 9,2±3,2 | NS | 224 | — | — |
| MORPHINE+ COMPOSE DE FORMULE (I) | 8 S.C. 30 S.C. | 37,1±7,8 | 0,001 | 904 | 0,01 | 337 |
| PETHIDINE | 40 I.P. | 5,1±0,4 | NS | 124 | — | — |
| COMPOSE DE FORMULE (I) | 20 I.P. | 6,5±0,7 | 0,10 | 158 | — | — |
| PETHIDINE+ COMPOSE DE FORMULE (I) | 40 I.P. 20 I.P. | 23±8,2 | 0,05 | 560 | 0,05 | 449 |

S = temps en secondes.
SE = écart type.
P = probabilité selon test t de Student.

La dose de 40 mg/kg intrapéritonéale de péthidine s'est montrée peu analgésique et d'une façon faiblement significative. L'association avec le composé de formule (I) fait apparaître l'effet attendu de la péthidine. Si à la 2ème exposition l'effet potentialisateur est peu significatif, en revanche la 3ème exposition révèle une forte potentialisation. Cette association se comporte donc de la même façon que l'association avec la morphine.

En conclusion, ces résultats montrent que le composé de formule (I), associé à la morphine ou à la péthidine, potentialise ou révèle l'effet analgésique des deux narcotiques étudiés.

## Propriétés Toxicologiques

L'administration de l'association dérivé morphinique et composé de formule (I) n'a pas révélé de manifestation toxique de quelque nature que ce soit comparativement d'une part à l'administration des 2 constituants pris séparément et d'autre part à l'administration de la morphine avec d'autres substances inhibitrices d'uptake de 5-HT de structure chimique différente comme la chlorimipramine.

## Utilisation Thérapeutique

La composition selon l'invention peut être utilisée en thérapeutique humaine sous forme de solutés injectables, suppositoires, comprimés . . . pour le traitement des états hyperalgiques.

La posologie journalière peut être une composition renfermant entre 0,0025 mg/kg et 125 mg/kg du dérivé morphinique et 0,01 mg/kg et 500 mg/kg du composé de formule (I).
Exemples de formes pharmaceutiques.

1) Solutés injectables

— Formule 1:

| | |
|---|---|
| Morphine, chlorhydrate | 10 mg |
| Composé de formule (I) | 10 mg |
| Acide ascorbique | 12 mg |
| Métabisulfite de sodium | 10 mg |
| Chlorure de sodium | 10 mg |
| Eau pour préparation injectable q.s.p. | 2 ml |

— Formule 2:

| | |
|---|---|
| Morphine, chlorhydrate | 20 mg |
| Composé de formule (I) | 50 mg |
| Acide ascorbique | 60 mg |
| Métabisulfite de sodium | 25 mg |
| Chlorure de sodium | 10 mg |
| Eau pour préparation injectable q.s.p. | 5 ml |

— Formule 3:

| | |
|---|---|
| Péthidine, chlorhydrate | 10 mg |
| Composé de formule (I) | 10 mg |
| Acide ascorbique | 12 mg |
| Métabisulfite de sodium | 10 mg |
| Chlorure de sodium | 10 mg |
| Eau pour préparation injectable q.s.p. | 2 ml |

— Formule 4:

| | |
|---|---|
| Péthidine, chlorhydrate | 50 mg |
| Composé de formule (I) | 50 mg |
| Acide ascorbique | 60 mg |
| Métabisulfite de sodium | 25 mg |
| Chlorure de sodium | 10 mg |
| Eau pour préparation injectable q.s.p. | 5 ml |

— Formule 5:

| | |
|---|---|
| Péthidine, chlorhydrate | 100 mg |
| Composé de formule (I) | 100 mg |
| Acide ascorbique | 120 mg |
| Métabisulfite de sodium | 50 mg |
| Chlorure de sodium | 20 mg |
| Eau pour préparation injectable q.s.p. | 10 ml |

Le procédé de préparation de ces 5 formules de solutés injectables est le suivant: Dissoudre les constituants dans de l'eau dégazée par ébullition et refroidie ou par barbotage à l'azote. Répartir en ampoules sous atmosphère d'azote. Stériliser les ampoules à 100°C pendant une heure à l'autoclave.

Les solutés peuvent être stérilisés par filtration et répartis stérilement. Les ampoules sont à l'abri de la lumière.

2) Suppositoires

— Formule 1:

| | |
|---|---|
| Péthidine, chlorhydrate | 10 mg |
| Composé de formule (I) | 10 mg |
| Butylhydroxyanisol | 2 mg |
| Glycéride semi-synthétique q.s.p. | 2 g |

— Formule 2:

| | |
|---|---|
| Péthidine, chlorhydrate | 50 mg |
| Composé de formule (I) | 25 mg |
| Butylhydroxyanisol | 2 mg |
| Glycéride semi-synthétique q.s.p. | 2 g |

— Formule 3:

| | |
|---|---|
| Péthidine, chlorhydrate | 100 mg |
| Composé de formule (I) | 50 mg |
| Butylhydroxyanisol | 2 mg |
| Glycéride semi-synthétique q.s.p. | 2 g |

— Formule 4:

| | |
|---|---|
| Péthidine, chlorhydrate | 100 mg |
| Composé de formule (I) | 100 mg |
| Butylhydroxyanisol | 2 mg |
| Glycéride semi-synthétique q.s.p. | 2 g |

Le procédé de préparation de ces 4 formules de suppositoires est le suivant:
Dissoudre le BHA (butylhydroxyanisol) dans la masse d'excipient fondu. Ajouter le chlorhydrate de péthidine puis le composé de formule (I). Mélanger. Homogénéiser à l'aide d'un moulin colloïdal ou d'un homogénéisateur à cisaillement. Couler dans des moules de 2 grammes. Refroidir.

**Revendications**

1. Composition analgésique caractérisée en ce qu'elle comprend un composé de formule (I):

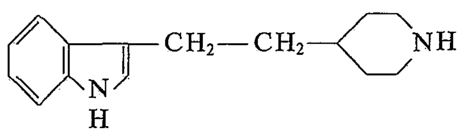

(I)

et la morphine ou un dérivé morphinique.

2. Composition analgésique selon la revendication 1 dans laquelle la proportion en poids de la morphine ou du dérivé morphinique varie de 0,1 à 10 fois le poids du composé de formule (I).

**Patentansprüche**

1. Analgetisch wirksame Zubereitungen, dadurch gekennzeichnet, daß sie eine Verbindung der allgemeinen Formel (I)

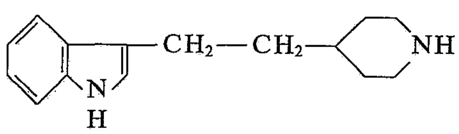

(I)

und Morphin oder ein Morphinderivat enthalten.

2. Analgetisch wirksame Zubereitungen gemäß Anspruch 1, dadurch gekennzeichnet, daß der Gewichtsanteil des Morphins oder des Morphinderivates das 0,1- bis 10fache des Gewichts der Verbindung der Formel (I) beträgt.

**Claims**

1. Analgesic composition which comprises a compound of formula (I):

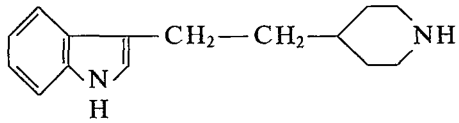

(I)

and morphine or a morphine derivative.

2. Analgesic composition according to claim 1 where in the proportion by weight of morphine or morphine derivative varies from 0,1 to 10 times the weight of the compound of formula (I).